(19)

Europäisches
Patentamt

European
Patent Office

Office européen
des brevets

(11) **EP 4 467 957 A1**

(12) **EUROPEAN PATENT APPLICATION**

(43) Date of publication:
**27.11.2024 Bulletin 2024/48**

(21) Application number: **23175071.2**

(22) Date of filing: **24.05.2023**

(51) International Patent Classification (IPC):
**G01M 11/00** (2006.01)    **A61B 34/20** (2016.01)
**G01B 11/16** (2006.01)    **H04B 10/00** (2013.01)
**H04J 14/00** (2006.01)

(52) Cooperative Patent Classification (CPC):
**G01M 11/3172; A61B 34/20; A61B 90/30;**
**G01L 1/242; G02B 6/274; G02F 1/0136;**
**G02F 1/212; G02F 1/31; H04B 10/00; H04J 14/00;**
A61B 2034/2061; A61B 2090/306

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **VAN DER GRAAFF, Leon**
  **Eindhoven (NL)**

• **EFIMOV, Mikhail**
  **Eindhoven (NL)**
• **SCHLEIPEN, Johannes Joseph Hubertina**
  **Barbara**
  **5656AG Eindhoven (NL)**
• **HORIKX, Jeroen Jan Lambertus**
  **Eindhoven (NL)**

(74) Representative: **Philips Intellectual Property &**
**Standards**
**High Tech Campus 52**
**5656 AG Eindhoven (NL)**

(54) **OPTICAL NETWORK WITH POLARIZATION DIVERSITY**

(57)    The present invention relates to an optical network, comprising a fiber optic path (12). The fiber optic path (12) comprises a first fiber optic branch (14) and a second fiber optic branch (16) separate from the first fiber optic branch (14), at least one optical switch (18) to toggle between input of light into the first fiber optic branch (14) and input into the second fiber optic branch (16), a polarization beam combiner (20) on an output side of the first and second fiber optic branches (14, 16), and at least one polarization controller (24, 26; 70) for controlling polarization in the fiber optic path (12). The optical network further comprises an optical power measuring modality (32) for measuring the optical output power of the polarization beam combiner (20). The optical network may be used in OFDR. Further, an OFDR system, an OSS system and a method of creating time-multiplexed orthogonal polarization states of light in a fiber optic network (10) are described.

FIG.1

EP 4 467 957 A1

**Description**

FIELD OF THE INVENTION

The present invention generally relates to optical networks with polarization diversity. In particular, the present invention relates to an optical network, in particular for use in Optical Frequency Domain Reflectometry (OFDR). Further, the present invention relates to a system for OFDR, further to an optical shape sensing system, and still further to a method of creating time-multiplexed orthogonal polarization states of light in a fiber optic network.

BACKGROUND OF THE INVENTION

Optical Frequency Domain Reflectometry (OFDR) requires the cancellation of birefringent effects in a sensing fiber. For this, it must be interrogated with light that has orthogonal states of polarization.

An exemplary field of application of OFDR is Fiber Optic Real Shape (FORS), which may also be referred to as optical shape sensing (OSS). FORS is a technique for visualizing the three-dimensional shape of medical devices such as catheters and guidewires. Shape is determined from the analysis of measured strains in a 'sensor' optical fiber which is embedded in the device. The sensor fiber typically is a specially crafted four-core optical fiber. The four cores allow measuring bend (in two degrees of freedom), twist and temperature along the sensor fiber. One core may be centrally placed in the optical fiber. The other three may be wound helically around the center core. Now, bend of the sensor fiber will introduce a linear strain profile over the cross-section of the sensor at the location of the bend. Twist will shorten or lengthen the outer cores, depending on the direction of the twist, while the center core is unaffected. Temperature and/or tensile strain will impact all cores. All cores may have a low intensity fiber Bragg grating (FBG) in the refractive index such that the sensor fiber is weakly scattering over the full length.

The strain in the sensor fiber is found by measuring optical path lengths and comparing these path lengths to a reference, in which the sensor fiber has a known shape, e.g., straight. This known shape is typically measured in a calibration procedure during manufacturing. OFDR is used for distributed sensing of these path lengths. The wavelength sensitive structures, e.g. the FBG sensors are included in the measurement (sensor) arm of the arms of the interferometer. Scanning the laser wavelength gives rise to beating frequencies on the detectors of the measurement interferometers in the interrogator. The beating frequency corresponds to the unbalancing of the interferometer, i.e., the optical path length difference between the measurement and reference arm. By means of a Fourier transform, the signal from different points on the sensor can be separated. The phase of the beating signal (for every point) is a very sensitive measure for optical path length difference but has the limitation of being wrapped, i.e., being measured modulo $2\pi$. The change in optical path length difference between points on the sensor fiber when the shape signals are compared to the reference signal is a measure for the accumulated strain between those points.

A bent optical fiber exhibits birefringence, i.e., the refractive index depends on the state of polarization (SOP) of the light propagating through the fiber. As a result, the optical path length measured using OFDR is depending on the polarization of the light fed into the sensor fiber and the shape of the sensor fiber. This is an undesired effect, as it impairs the comparison with the reference shape. A solution for this is to use a so-called polarization diversity scheme. The sensor is interrogated using two orthogonal states of polarization. Provided that polarization dependent loss (PDL) does not play a significant role, the orthogonality will be conserved throughout the optical path. Now, the averaged optical path length is independent of the input polarization and birefringence of the sensor.

Current FORS systems use an electro-optic polarization controller to toggle between two polarization states. This type of controller has several advantages. The speed of the controller allows a high frame rate (60 Hz), required by the clinical need of visualizing shapes to a physician at video speed. The controller has a low polarization mode dispersion (PMD) and has therefore the ability of making an orthogonal polarization across the whole wavelength range (e.g., 1530-1560 nm) using three control elements, provided that there is low PMD at the input of the controller.

The use of an electro-optic polarization controller might also have some disadvantages. The main disadvantage is that this type of controller might be sensitive to sudden increases in insertion loss after ~1000 h of operation due to aging effects. Further, the response of the controller is very temperature sensitive. In practice, this requires (near) continuous monitoring of the polarization to guarantee orthogonality of the polarization states. Deviations from orthogonality cannot be observed directly in the OFDR data, and thus additional means of measuring must be present in the system.

A method for detecting deviations of orthogonality is to use a polarization beam splitter (PBS) with two photodiodes. First, one state is aligned to minimum transmission of one of the detectors. After switching to the second state, the power on the second detector is a measure of deviation from orthogonality. In case of orthogonality, the power on the second detector will be minimum.

Piezo-based polarization controllers provide an alternative to electro-optical-based polarization controllers for creating polarization diversity. However, these suffer from a slower response, long term drift, hysteresis, and an inherent PMD.

PMD introduces a dependence of the polarization on the wavelength of the light. Therefore, creating two states of polarization that are orthogonal over the whole wavelength range becomes more complicated. Creating orthogonal states will require more degrees of freedom of controlling the polarization. This dependence of polarization on wavelength also impairs observing deviations from orthogonality using a PBS, as it is not possible to create a state which has minimum transmission on a photodetector over the whole wavelength range. Therefore, deviations from orthogonality have to be observed by different means, e.g., using a polarization analyzer.

WO 2004/005973 A2 discloses polarization diversity detection without a polarizing beam splitter. This document describes a fiber optic measurement device for use in OFDR that performs polarization diversity detection without using a polarizing beam splitter. Field vectors from one interferometer arm are used as the basis upon which to project a field vector from the other interferometer arm. The optical network uses a comparably large number of couplers, thus increasing the system costs.

There is still a need for improvements in controlling orthogonality of polarization states which overcome at least some of the above disadvantages.

## SUMMARY OF THE INVENTION

It is an object of the present invention to provide an optical network, in particular for use in OFDR, which is able to create orthogonal polarization states of light with the use of durable components which are resilient against rapid switching for extended periods of time.

It is a further object of the present invention to provide an optical network which is able to create inherently orthogonal polarization states of light with no need for detecting the state of polarization.

It is a further object of the present invention to provide an optical network which is able to create orthogonal polarization states of light with relaxed requirements on the speed and accuracy of polarization controllers.

It is still a further object of the present invention to provide a system for Optical Frequency Domain Reflectometry.

It is still a further object of the present invention to provide an optical shape sensing system.

It is still a further object of the present invention to provide a method of creating time-multiplexed orthogonal polarization states of light in a fiber optic network.

In a first aspect of the present invention, an optical network for use in Optical Frequency Domain Reflectometry is provided, comprising:

a fiber optic path comprising:

a first fiber optic branch and a second fiber optic branch separate from the first fiber optic branch,
at least one optical switch to toggle between input of light into the first fiber optic branch and input into the second fiber optic branch,
a polarization beam combiner on an output side of the first and second fiber optic branches,
at least one polarization controller for controlling polarization in the fiber optic path,

an optical power measuring modality for measuring the output power of the polarization beam combiner.

The optical network according to the invention provides a new polarization diversity scheme which may be briefly referred to as 'Polarization Diversity by Switching and Combining'. Light input is toggled between first and second fiber optic branches via at least one optical switch. That is, light is alternatingly input into the first fiber optic branch and into the second fiber optic branch. The at least one optical switch may toggle the input into the first and second fiber optic branches in synchronization with wavelength scans of the light. For example, light of a first wavelength scan is input only into the first fiber optic branch and light of a subsequent second wavelength scan is input only into the second fiber optic branch, light of a subsequent third wavelength scan is input only into the first fiber optic branch, and so on. Light from the first fiber optic branch and from the second optic branch is fed into a polarization beam combiner (PBC). The PBC may be an optical device with a first input port, a second input port, and one output port. The first fiber optic branch and the second fiber optic branch are connected to the PBC. The first fiber optic branch may be connected to the first input port of the PBC, and the second fiber optic branch may be connected to the second input port of the PBC. The output port may be connected to a single fiber of an interferometer, e.g. of an OFDR system. The first input port may provide minimum insertion loss for a first polarization state and maximum insertion loss for a second polarization state orthogonal to the first polarization state. The second input port may provide minimum insertion loss for the second polarization state and maximum insertion loss for the first polarization state. If the PBC behaves ideally, the PBC will only transmit the polarization state that is aligned to the respective port. In practice, the PBC may have a finite extinction ratio. At the output port of the PBC, the transmitted polarization states are inherently orthogonal or very close to orthogonal. For example, if the light output from the first fiber optic branch has a first polarization state aligned with the first input port of the PBC, this polarization state will be transmitted by the PBC. If the light output from the first fiber optic branch has a polarization state orthogonal to the first polarization state, this polarization state will not be transmitted by the PBC. If the light output from the second fiber optic branch has a second polarization state aligned with the second input port of the PBC, this polarization state will be transmitted by the PBC. If the light output from the second fiber optic branch has a polarization state orthogonal to the second polarization state, this polarization state will be transmitted by the PBC. The polarization at the output of the polarization beam combiner now toggles between two polarization states. Due to the optical properties of the polarization beam combiner these states have (very close to) orthogonal polarizations. Thus, as an advantage, there is no need for detecting the state of the polarization.

Polarization of the light in the fiber optic path is controlled by at least one polarization controller. The polarization controller has the function of setting the polarization state of the light fed into the polarization controller. Setting the polarization state may include changing the polarization state from an undefined state to a defined state or from a first defined state to a second defined state different from the first state. As will be described later, the at least one polarization controller may be arranged at various positions in the optical fiber path.

The optical power measuring modality for measuring the output power of the polarization beam combiner of the optical network according to the invention may be used to control the at least one polarization controller for minimizing insertion loss at the polarization beam combiner. Measuring the optical power at the output of the polarization beam combiner may be made during manufacturing, maintenance, during system startup, at regular intervals, or using (semi-) continuous feedback during system operation. The power measuring modality may be integrated in the system the optical network forms part of. For example, the signal detector of an OFDR system may be used as the power measuring modality.

The optical network according to the invention has several advantages over the known optical networks. The only component that is actuated at high frequency (e.g. 60 cycles per second) is the at least one optical switch. There are optical switches, in particular MEMS-based optical switches, which are known for their durability and which are specified to switch about $10^9$ times before wearing out. The optical network according to the invention does not require a polarization controller that toggles between two polarization states with high speed. Thus, the optical network according to the present invention is able to be constructed with durable components and is therefore resilient against rapid switching for extended periods of time.

The optical network according to the invention works over a wide range of wavelengths, requires only very coarse input polarization alignment and does not need means for detecting deviations from orthogonality, is tolerant to a substantial amount of PMD, does not require components with a short lifetime, and requires only slow polarization control.

The optical network according to the invention is particularly advantageous in applications where fast toggling between two orthogonal polarization states is required. The optical network according to the invention is particularly advantageous for use in Optical Frequency Domain Reflectometry, in particular in optical shape sensing.

Preferred embodiments of the invention are defined in the dependent claims and are described herein.

The at least one polarization controller may be arranged in one of the first and second fiber optic branches. If the at least one polarization controller is arranged in one of the first and second fiber optic branches, the polarization state can be well aligned with at least one input port of the polarization beam combiner. If the at least one polarization controller comprises only one polarization controller, e.g. arranged in one of the first and second fiber optic branches, or upstream of the at least one optical switch, cost reduction due to a lower number of controllers may be achieved.

It is particularly preferred, if the at least one polarization controller comprises a first polarization controller arranged in the first fiber optic branch and a second polarization controller arranged in the second fiber optic branch. With this arrangement, polarization of the light can be well controlled in both, the first and second fiber optic branches, in particular controlled for minimum insertion loss at the polarization beam combiner, or in other words, for maximum transmission of the corresponding polarization state through the polarization beam combiner. For example, if the first fiber optic branch is connected to the 's'-port of the polarization beam combiner, and the second fiber optic branch to the 'p'-port of the polarization beam combiner, the polarization controller in the first fiber optic branch is controlled for 's'-polarization, and the polarization controller in the second fiber optic branch is controlled for 'p'-polarization. A further advantage of this embodiment is that both controllers can be optimized and controlled independently from one another.

It is also possible that the at least one polarization controller comprises a first polarization controller arranged in one of the first and second fiber optic branches, and a second polarization controller arranged upstream of the optical switch.

In this case, the polarization controller in one of the fiber optic branches cannot be controlled or optimized independent from the polarization controller in front of the optical switch. In this case, both polarization controllers may be optimized sequentially.

The at least one polarization controller may be a mechanical controller that relies on bending the optical fiber (also called 'bat ear', or 'paddle controller'), an electro-optic polarization controller, or a piezo-based polarization controller. As described above, the optical network according to the invention requires only slow polarization control. Polarization control may only be needed on the time scale of external influences on the system and not at the frame rate of the system. The polarization controller(s) used in the optical network according to the invention should have sufficiently low PMD to be able to control the polarization over the desired wavelength range to minimize losses.

Preferably, the at least one optical switch is a MEMS-based optical switch. MEMS-based optical switches are advantageous because of their high durability and high speed switching capabilities.

Preferably, the at least one optical switch comprises one bipolar optical switch connecting to both the first and second fiber optic branches. In this embodiment, the optical switch has one input port and two output ports, the first of the two output ports connects to the first fiber optic branch and the second of the two output ports connects to the second fiber optic branch.

Alternatively, the at least one optical switch comprises a first unipolar optical switch connecting to the first fiber optic branch and a second unipolar optical switch connecting to the second fiber optic branch. The advantage of this embodiment is that only unipolar optical switches are needed which reduces costs. In this arrangement, a splitter in the fiber optic path in front of two unipolar optical switches may be needed to branch the light to the two unipolar optical switches.

As already mentioned above, it is possible and especially preferred in case the optical network is configured for use in OFDR, that the at least one optical switch is synchronized with wavelength scans of light emitted by a light source such that the wavelength scans alternate at the output of the PBC with mutually orthogonal polarization states.

Further preferably, the at least one polarization controller may be configured to set the polarization based on the measured output power of the polarization beam combiner. Thus, the polarization controller may set the polarization state for maximum output power at the output port of the PBC, or in other words, for minimum insertion loss at the polarization beam combiner. To this end, the optical power measuring modality may be configured to control the at least one polarization controller.

In a further aspect of the present invention, a system for optical for Optical Frequency Domain Reflectometry is provided, comprising:

a light source for emitting light, and
an optical network according to the first aspect into which light emitted by the light source is fed.

Preferably, the light source is configured to trigger the at least one optical switch according to wavelength scans of the light.

According to a still further aspect, an optical shape sensing (OSS) system is provided, comprising a system according to the previous aspect and an elongated optical fiber connected to the optical network for interrogating the optical fiber. The optical network according to the invention may be part of the interrogator of the OSS system.

The elongated optical fiber may be incorporated into a shape sensing enabled device, for example a shape sensing enabled medical device, like a guidewire or catheter. The light output from the polarization beam combiner may be fed into the elongated optical fiber of the shape sensing enabled device.

According to a still further aspect of the present invention, a method of creating time-multiplexed orthogonal polarization states of light in a fiber optic network is provided, comprising:

inputting a light beam into a fiber optic path, the fiber optic path comprising a first fiber optic branch and a second fiber optic branch separate from the first fiber optic branch,
toggling between input of light into the first fiber optic branch and input of light into the second fiber optic branch,
controlling polarization of the light in the fiber optic path,
inputting the light from the first and second fiber optic branches into a polarization beam combiner,
measuring an optical output power of the polarization beam combiner.

It shall be understood that the claimed systems and the claimed method have similar and/or identical preferred embodiments as the claimed optical network, in particular as defined in the dependent claims and as disclosed herein.

BRIEF DESCRIPTION OF THE DRAWINGS

These and other aspects of the invention will be apparent from and elucidated with reference to the embodiments described hereinafter. In the following drawings:

Fig. 1 shows an optical layout of an optical network according to an embodiment;
Fig. 2 shows an optical layout of a measurement setup for finding the effect of a finite extinction ratio on polarization;
Fig. 3a and 3b show graphs, wherein the graph in Fig. 3a reveals the dependency of transmission of the PBC on the angle of the incoming polarization to the axis of maximum transmission of the PCB, and the graph in Fig. 3b shows the relation of the angle between the polarization state at the exit of the PBC and the PBC's axis of maximum transmission and the angle between the polarization state at the entrance of the PBC and the PBC's axis of maximum transmission;

Fig. 4 shows an interferometric experimental setup to verify the orthogonality of the 'p'- and 's'-port of the PBC;

Fig. 5a and 5b show graphs revealing the result of interferometric method for verification of orthogonality of the light transmitted by the 'p'- and 's'-arms of the optical network, wherein the graph in Fig. 5a shows the result when both arms are aligned for maximum transmission through the PBC, and the graph in Fig. 5b shows the result when one arm is aligned for optimum transmission, the other for minimum transmission;

Fig. 6a and 6b show optical layouts of alternative embodiments of the optical network;

Fig. 7a and 7b show optical layouts of further alternative embodiments of the optical network;

Fig. 8 shows an optical layout of a further alternative embodiment of the optical network;

Fig. 9 shows an optical layout of a further alternative embodiment of the optical network;

Fig. 10 shows a block diagram of an OFDR system; and

Fig. 11 shows a block diagram of an optical shape sensing system.

DETAILED DESCRIPTION OF THE INVENTION

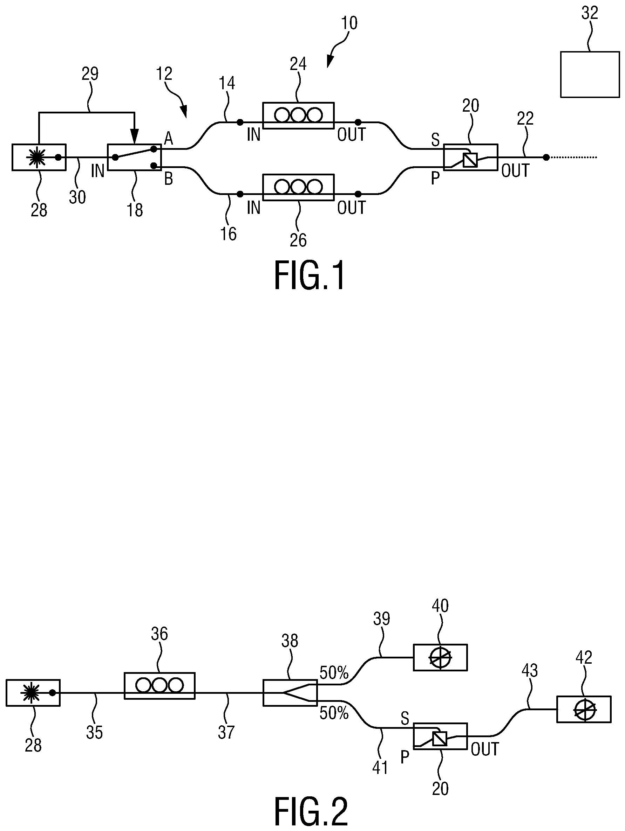

Fig. 1 shows a sketch of an optical layout of an optical network 10 according to the present disclosure. The optical network 10 provides polarization diversity by switching and combining (in the following referred to as PDSC). The optical network 10 comprises a fiber optic path 12. The fiber optic path 12 is built by one or more optical fibers. The fiber optic path 12 comprises a first fiber optic branch 14 and a second fiber optic branch 16 separate from the first fiber optic path 14. Each of the first fiber optic branch 14 and the second fiber optic branch 16 may comprise a single optical fiber. The fiber optic path 12 further comprises at least one optical switch 18 which here is a single optical switch. The optical switch 18 is a bipolar optical switch, i.e. the switch has an input port IN and two output ports A and B. The output port A is connected to the first fiber optic branch 14 and the output port B is connected to the second fiber optic branch 16. The optical switch 18 is configured to toggle between input of light into the first fiber optic branch 14 and input of light into the second fiber optic branch 16. Preferably, the optical switch 18 is a MEMS-based fiber optic switch.

The fiber optic path 12 further comprises a polarization beam combiner (PBC) 20. The PBC 20 comprises two input ports denoted with 's' and 'p' and one output port denoted with 'OUT'. The 's'-port and 'p'-port of the PBC 20 in each case provide maximum transmission for a polarization state which is aligned with this port, and minimum transmission for a polarization state which is misaligned with this port, in particular which is orthogonal to the polarization state of maximum transmission. The first fiber optic branch 14 is connected to the s-port and the second fiber optic branch 16 is connected to the p-port of the PBC 20. The output port OUT of the PBC 20 may be connected to a single optical fiber 22 which may be part of or connected to an interferometer.

The fiber optic path 12 further comprises at least one polarization controller. In the present embodiment, the at least one polarization controller comprises a first polarization controller 24 arranged in the first fiber optic branch 14 and a second polarization controller 26 arranged in the second fiber optic branch 16. The polarization controller 24 and 26 may be mechanical controllers that rely on bending the optical fiber of the respective fiber optic branch 14 and 16 (also called -bat ear', or paddle controller'), electro-optic controllers, or piezo-based polarization controllers. Each polarization controller 24 and 26 has an input port denoted with IN and an output port denoted with OUT. The output of both polarization controllers 24 and 26 is fed to the PBC 20.

Fig. 1 further shows a light source 28. The light source 28 especially is a wavelength tunable light source. The light source may be a laser, especially a wavelength tunable laser. The light source 28 may emit light in a range of wavelengths, for example, a range of 30 nm width. As an example, the wavelength range may be from 1515 nm-1545 nm. The light source 28 may form part of the optical network 10 or may be a separate modality. The light emitted by the light source 28 is fed to the optical network 10 via a single optical fiber 30 connected to the input port IN of the optical switch 18.

In operation of the optical network 10, light from the light source 28 is fed to the optical switch 18. The optical switch 18 may switch between the output ports A and B in synchronization with wavelength scans of the light source 28 such that after each wavelength scan light output from the switch 18 changes from output port A to output port B and vice versa. Thus, light input into the branches 14 and 16 may alternate after each wavelength scan. In Fig. 1, a line 29 indicates the light source trigger used for toggling of the optical switch 18 synchronous with wavelength scans of the light source 28. After each wavelength scan of the light source 28, the light source 28 triggers the switch 18 to change the light input into the other of the fiber optic branches 14 and 16.

Light input into the PBC 20 thus toggles between the two input ports 's' and 'p' of the PBC 20. As a result, the polarization at the exit of the PBC 20 toggles between two polarization states. Due to the optical properties of the PBC 20, these states have (very close to) orthogonal polarizations.

The optical network 10 further comprises an optical power measuring modality 32 for measuring the optical output power of the PBC 20. One or more output power signals of the optical power measuring modality 32 may be used to control the polarization controllers 24 and 26 for setting the polarization states in the branches 14 and 16 for optimum alignment with the 's'- and 'p'-port of the PBC 20, respectively.

The characteristics of the components of the optical network 10 described above and further below will be described in more detail hereinafter.

A polarization beam combiner (PBC) like PBC 20 is an optical device with two input ports, denoted with 's' and 'p' and one output port denoted with 'OUT'. The labels 's' and 'p' are used here as a matter of convention, although it does not have a well-defined meaning in a fiber optic system. However, 's' and 'p' here mean polarization states which are orthogonal to each other. An ideal PBC will only transmit the polarization state that is aligned to the respective input port: The s-port will transmit s-polarized light and absorb or dissipate p-polarized light and vice-versa for the p-port. The p- and s-polarization states are, by definition, orthogonal to each other.

In practice, PBCs have a finite extinction ratio. That means that the transmission will not go to zero for a misaligned polarization, e.g., s-polarization fed into the p-port. The ratio between maximum and minimum transmission is called the extinction ratio $\rho$ and is conveniently expressed in dB. The transmission of a PBC with finite extinction ratio and neglecting all nominal losses is given by Malus law:

$$T = \frac{1 + \Gamma \cos(\phi_{in})}{1 + \Gamma}, \qquad (1)$$

where $\phi_{in}$ expresses the angle of the incoming polarization to the axis of maximum transmission of the PBC. The angle $\phi_{in}$ is measured on the Poincaré sphere (deg pc) and is zero for perfect alignment and 180° for perfect misalignment (polarization states which are orthogonal to one another are represented by vectors with opposite direction which lie on a diameter of the Poincaré sphere and thus enclose an angle of 180°). The 'unit loss' $\Gamma$ has a value between 0 and 1 and is related to the extinction ratio $\rho$ by

$$\frac{1 - \Gamma}{1 + \Gamma} = 10^{-\frac{\rho}{10}}. \qquad (2)$$

Next, the effect of a finite extinction ratio on the polarization state of the light at the output of the PBC 20 will be described with reference to Figs. 2 to 5. The impacts of the finite PBC extinction ratio on the state of polarization at the output OUT of the PBC 20 was tested by two experiments.

Fig. 2 shows a first measurement setup for finding the effect of a finite extinction ratio on polarization at the output port OUT of the PBC 20. The measurement setup comprises a light source like light source 28 in Fig. 1, e.g. a laser. The light source 28 is connected to a polarization controller 36 via an optical fiber 35, and the polarization controller 36 is connected to a splitter 38 via an optical fiber 37. The splitter 38 is a 50-50 splitter. One output port of the splitter 38 is connected via an optical fiber 39 to a first polarization analyzer 40. The output port of the splitter 38 is connected via an optical fiber 41 to one of the input ports ('s' or 'p') of the PBC 20. The output port OUT of the PBC 20 is connected to a second polarization analyzer 42 via an optical fiber 43.

The first experiment investigates the effect of misaligning the input polarization to the PBC 20. Light of the light source 28 is fed into one of the input ports (in Fig. 2 the 's'-port) of the PBC 20. The polarization at both input and output of the PBC 20 is measured using the first and second polarization analyzers 40 and 42.

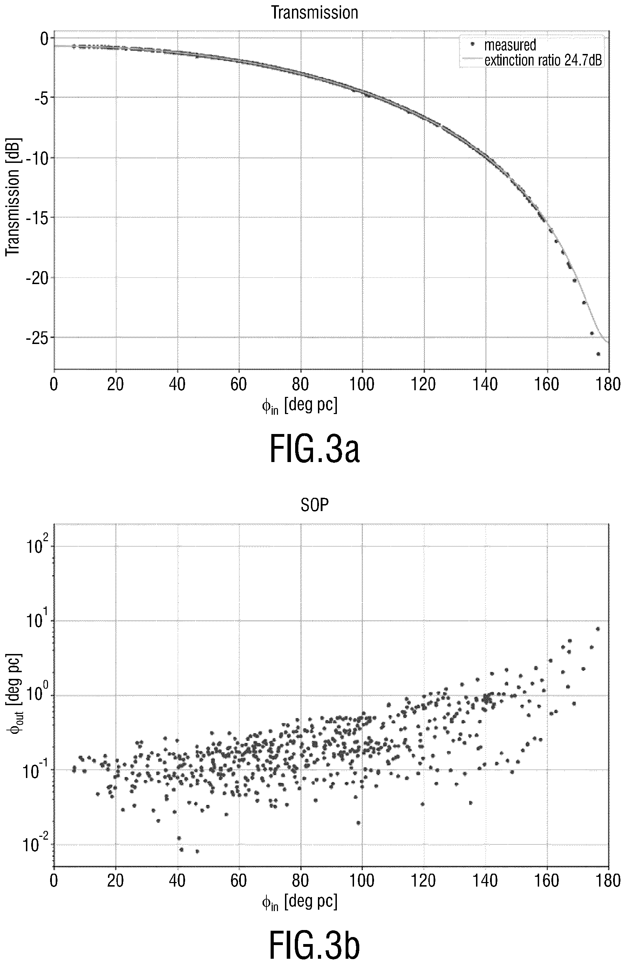

The PBC 20 is fed with a wide range of polarization states. Fig. 3a shows the measured relation between $\phi_{in}$ (defined above) and the transmission through the PBC 20. Malus law (equation 1) provides a good description of the data and fitting yields $\rho$ = 24.7dB.

Let $\phi_{out}$ be the angle (in units deg pc) between the state of polarization (SOP) at the exit or output port OUT of the PBC 20 and the PBC's axis of maximum transmission, then the relation between $\phi_{in}$ and $\Phi_{out}$ is shown in Fig. 3b. The output SOP appears to be within a few degrees of the maximum transmission axis regardless the polarization state entering the PBC 20. The angle $\phi_{out}$ does increase somewhat for high values of $\phi_{in}$. When $\phi_{in}$ approaches 180° (maximum misalignment) a deviation of up to 10° is observed. However, at these high angles the light experiences very low transmission. Therefore, the measured increase in output angle $\phi_{out}$ might suffer from a bias originating from offsets or non-linearities in the polarization analyzer 42 at low signal levels. It may be therefore concluded that the finite extinction ratio does not, or hardly, impact the output polarization of the PBC 20.

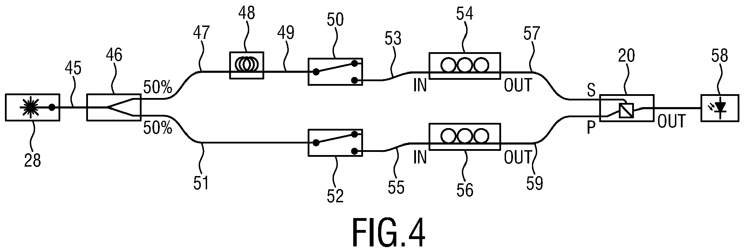

The second experiment was performed with a measurement setup shown in Fig. 4 to verify that the light transmitted by the p- and the s-port is orthogonal. The measurement setup shown in Fig. 4 comprises the light source 28 connected via an optical fiber 45 to a splitter 46, here a 50-50 splitter. One output port of the splitter 46 is connected to a delay line 48 which in turn is connected via an optical fiber 49 to a first optical switch 50. The other output port of the splitter 46 is connected to a further optical switch 52 via an optical fiber 51. The output ports of the switches 50 and 52 are connected to input ports IN of polarization controllers 54 and 56 via optical fibers 53 and 55, respectively. The output ports OUT of the controllers 54 and 56 are connected to the s-port and the p-port of PBC 20 via fibers 57 and 59, respectively. The PCB 20 is connected to a detector 58. Thus, the PBC 20 is fed by light from light source 28, which may be a single scanning laser source. The delay line 48 provides an intentional arm length difference to the p and s-ports of the PBC 20. The output port OUT of the PBC 20 is connected to the photodetector 58. This setup exploits the fact that orthogonal polarizations do not interfere, while deviations from orthogonality will result in a beating signal on the photodetector 58. The signal $D(t)$ is given by:

$$D(t) = \left|E_p\right|^2 + |E_s|^2 + 2\left|E_p\right||E_s|\cos(\theta/2)\cos(\omega t + \phi), \qquad (3)$$

where $|E_p|^2$ and $|E_s|^2$ are the power transmitted by the p- and s-port respectively, $\theta$ the angle between the state of polarization vectors on the Poincaré sphere of the light transmitted by the p- and s-port, and $\cos(\omega t + \phi)$ an interference term with a beat frequency $\omega$, which is proportional to the interferometer arm length difference (the delay) and the laser scan speed.

To measure $\theta$, three measurements were performed with the measurement setup in Fig. 4. During the first measurement, the p-arm is disconnected using the fiber optic switch 52. That is, light is not fed from the light source 28 to the p-port of the PBC 20. The recorded value on the photodetector 58 is thus equal to the power in the s-arm, $D_1(t) = |E_s|^2$. In a second measurement, the power in the other arm is measured, i.e. optical switch 50 is open and optical switch 52 is closed. Thus, in the second measurement $D_2(t) = |E_p|^2$ is measured. Finally, both switches 50 and 52 are closed so that both arms are connected to measure $D_3(t)$ such as given in equation 3 above. A band pass filter BPF around $\omega$ and envelope tracking by means of a Hilbert transform is used to obtain a magnitude of the beating signal $b = \text{Envelope}\{\text{BPF}\{D_3(t)\}\}$. Now $\theta$ is obtained using

$$\theta = 2\arccos\frac{b}{2|E_e||E_o|}. \qquad (4)$$

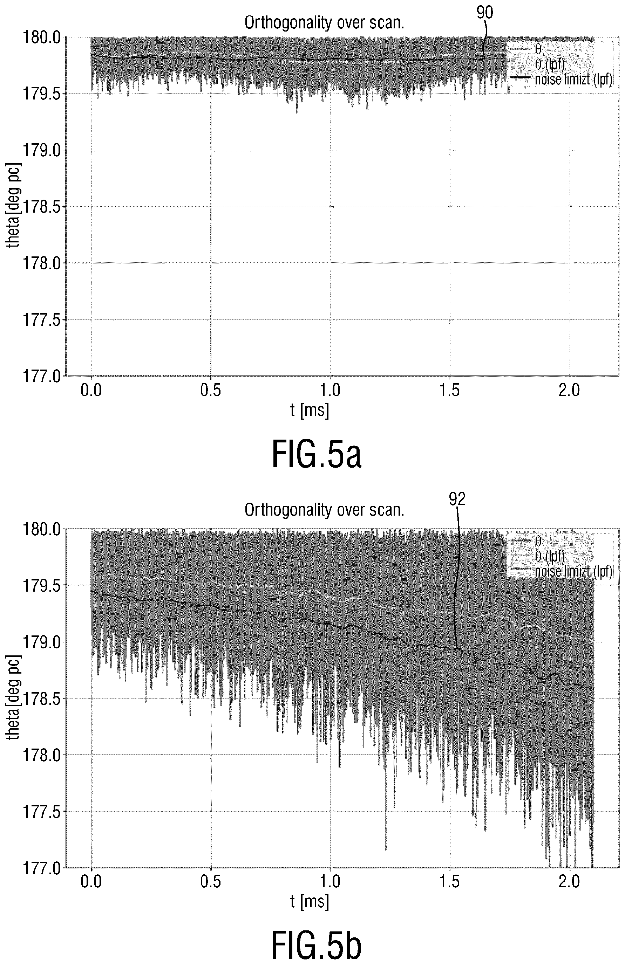

Fig. 5a and 5b show the results of the measurements. Fig. 5a shows the case in which both arms are aligned for maximum transmission through the PBC 20. The angle $\theta$ is measured to be larger than 179.7°. This measurement is limited by the noise from the light source 28 that passes through the bandpass filter. An estimate of the impact of this noise is displayed in Fig. 5a by a curve 90. It shows that the measured deviation from orthogonality can be attributed to the noise.

The interferometric setup is also used to verify the result of the orthogonality of the SOP at the output port OUT of the PBC 20 being hardly impacted by the polarization alignment at the input ports s and p of the PBC 20. This is done by aligning one of the arms in Fig. 4 to minimum transmission, thus having a strong misalignment, while the other branch is aligned for maximum transmission. The result of this measurement is shown in Fig. 5b. The angle between the s and p-branch is determined to be greater than 179°. Again, the measured deviation from orthogonality can be explained by the noise. An estimation of the impact of noise is also displayed in Fig. 5b by a curve 92.

In conclusion, the SOP of the light transmitted by the p- and s-port of the PBC 20 are observed to be orthogonal for practical purposes, even in the presence of a finite PBC extinction ratio, and regardless of any misalignment of the SOP at the input of the PBC.

Another advantage of PDSC according to the present disclosure is that it works over a wide range of wavelengths. Most polarization controllers rely on the introduction of birefringence in an optical element, such as an optical fiber. As a result, the resulting polarization change will depend on the wavelength. This effect becomes even more severe when the polarization controller relies on a slight modulation of a strong nominal birefringence, such as in piezo- or thermal-based polarization controllers in which polarization is controlled by modulating a large nominal birefringence. In contrast, the PBC 20 relies on loss rather than introducing optical path length differences for modifying the SOP and is therefore not wavelength dependent. Ultimately, the wavelength range is limited by the support of the individual components. If the switch 18 is a MEMS-based optical switch, it accepts a very wide wavelength range of ~400 nm at a nominal wavelength of 1550 nm. The PBC 20 accepts a range of 80 nm at a nominal wavelength of 1550 nm. This is much wider than needed for optical shape sensing, for example.

Another beneficial effect of PDSC is that it requires only very coarse input polarization alignment and does not need means of detecting deviations from orthogonality. PDSC creates inherently orthogonal polarization states as described above. It does, however, need alignment of the input polarization at the input ports s and p of the PBC 20 to minimize the insertion losses in the PBC 20. If, as preferred, the optical network 10 is used in a OFDR system, a control signal that can be used for controlling the polarization controllers 24 and 26 to set the input polarization in the branches 14 and 16 for minimum insertion loss by the PBC 20 can be the magnitude of the OFDR signal, already available in the OFDR system. No separate means for detecting orthogonality, e.g., a polarization analyzer, are required.

The required accuracy of alignment of the input polarization with the input ports of the PBC 20 depends on how much losses can be tolerated. These losses are given by Malus law (equation 1 above). For example, when a loss of 1 dB can be accepted, the alignment should be better than 54°; an accuracy better than 17° gives losses smaller than 0.1 dB.

Anyways, the constraints on alignment accuracy are orders of magnitude lower than the achieved deviations from orthogonality.

In the optical network 10, the optimization of the polarization outputs of polarization controllers 24 and 26 can be implemented using a numerical search method such as the Nelder-Mead algorithm. This has the advantage that minimal (if any) calibration of the polarization controllers 24 and 26 is required. Accompanying advantages are: a strong robustness against environmental, e.g., temperature, changes; when the optical network is used in FORS, optimization of the alignment does not interfere with the shape sensing, as orthogonality is maintained. Only a slight decrease of the signal-to-noise ratio might be observed when the transmission decreases.

Another advantage of PDSC is that it is tolerant to a substantial amount of polarization mode dispersion (PMD).

PMD forms a challenge for polarization alignment when working with wide band or scanning light sources like light source 28. A system that is well aligned for the center wavelength $\lambda_c$ might be misaligned for other wavelengths. PDSC is tolerant to some PMD, either present at the input or introduced by the polarization controllers 24, 26. Suppose the controllers are well aligned for $\lambda_c$, then the PMD will introduce a misalignment at the input ports s and p of the PBC 20 and therefore transmission losses in the PBC 20. The amount of PMD that can be tolerated depends on how much loss can be tolerated, analogous to the case of misalignment of the controllers 24, 26. Consider, for example, a system with center wavelength $\lambda_1$ = 1545 nm for which the PBC has maximum transmission, and a final wavelength $\lambda_2$ = 1555 nm. Suppose an insertion loss of 1 dB is acceptable, then the SOP for $\lambda_2$ may not deviate more than 17° on the Poincaré sphere from the SOP of $\lambda_1$. This corresponds to a maximum PMD of 0.012 ps. For a tolerated loss level of 0.1 dB, still 0.04 ps PMD is tolerated.

Another advantage of PDSC is that it does not require components with a short lifetime. A high frequent (60 cycles/sec) toggling between two polarization states can lead to failure of electro-optical polarization controllers. In contrast, in PDSC, the only component that is actuated at high frequency is the optical switch 18. MEMS-based optical switches are known for their durability and are specified to switch ~$10^9$ times before wearing out.

An accelerated life test was performed on ten fiber optic MEMS switches (sold by Sercalo Microtechnology Ltd.). The switches are actuated at 300 cycles per second, where one cycle is a switch back and forth. The switches have made more than $5 \cdot 10^9$ cycles without an apparent increase in insertion loss, without a significant change in switch time, and without missing transitions.

Least but not last, another advantage of PDSC is that it requires only slow polarization control.

Polarization control in PDSC is only needed on the timescale of external influences on the system and not at the frame rate of the system. The main source of polarization change originates from temperature changes. A rough estimate of the maximum amount of drift after start-up is 30° per hour. Given the course alignment required to minimize transmission losses, on the orders of tens of degrees, the polarization controllers 24 and 26 must be optimized a couple of times per hour. Optimizing typically takes on the order of 10 iterations. In conclusion, the polarization controllers 24 and 26 are expected to be actuated on average three orders of magnitude less frequent than the frame rate of the system, differently from prior art polarization diversity schemes. Apart from the advantages on lifetime, the low requirement on speed also makes room for significant cost reductions because of the wider range of types of polarization controllers PDSC is compatible with.

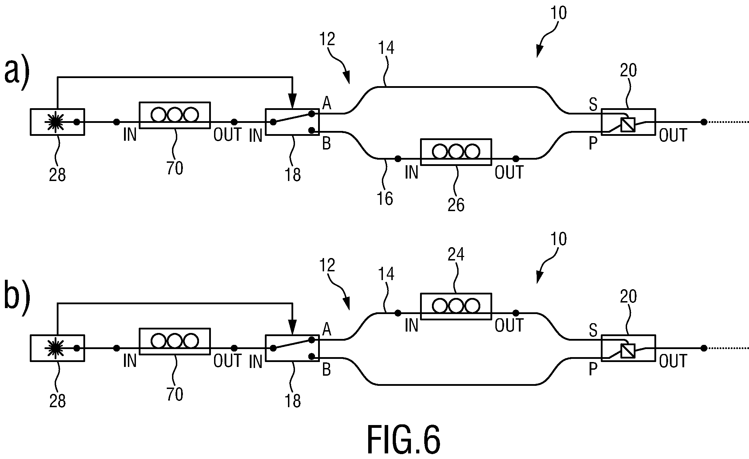

With reference to Figs. 6-9, further embodiments of optical networks will be described. Components or parts of the optical networks to be described below which are identical, similar or comparable with the components of the optical network 10 in Fig. 1 are labelled with the same reference numerals as in Fig. 1.

In the following, only the differences between the embodiments in Fig. 6 to 9 and the embodiment in Fig. 1 will be described. If not otherwise indicated, the previous description also holds for the following embodiments.

The optical power measuring modality 32 in Fig. 1 is omitted in Figs. 6 to 9, but may be present in all embodiments described below.

Fig. 6a and 6b show an optical network 10 in which only polarization controller 26 is arranged in the second fiber optic branch 60, while a second polarization controller 70 is arranged in the fiber optic path 12 upstream, i.e. before the optical switch 18. Fig. 6b shows an optical network 10 in which polarization controller 24 is arranged in the first fiber optic branch 14, and the second polarization controller 70 is arranged in the fiber optic path 12 before the optical switch 18. In both cases of Fig. 6a and 6b, the polarization controller 26 and the polarization controller 24, respectively, become dependent on the polarization controller 70 between the light source 28 and the optical switch 18. In such a case, polarization controllers 70 and 26 and 70 and 24, respectively, should be optimized sequentially.

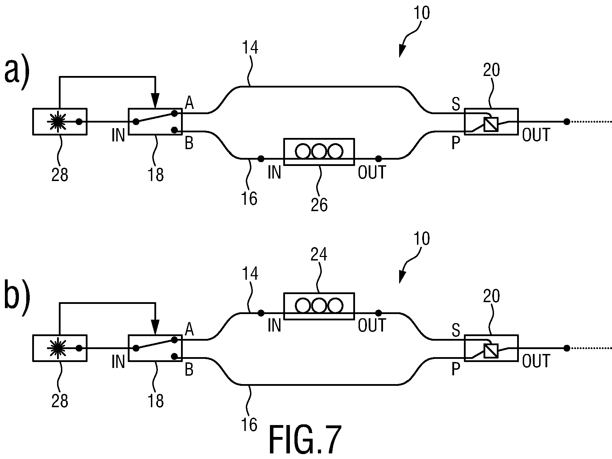

Fig. 7a and 7b show the optical network 10 with only one polarization controller, i.e. one polarization controller 26 in the second fiber optic branch 16 or polarization controller 24 in the first fiber optic branch 14. While control over polarization is reduced in the optical network 10 according to Fig. 7a and 7b, costs of these embodiments are reduced in comparison with Fig. 1 as only one polarization controller is used.

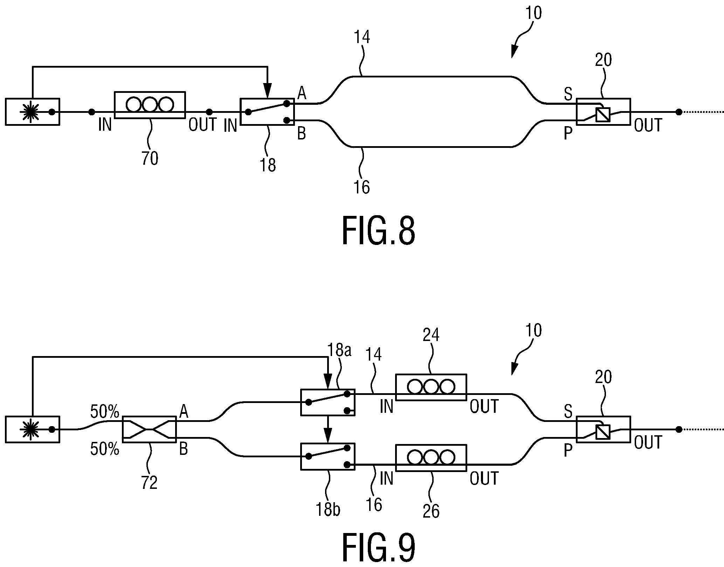

The optical network 10 according to Fig. 8 is similar to the embodiments in Fig. 7a and b, wherein the only polarization controller 70 is arranged before the optical switch 18. While again control over polarization is incomplete in comparison with the optical network 10 in Fig. 1, cost reduction is achieved because only one polarization controller is used.

In the optical network 10 according to Fig. 9, the bipolar optical switch 18 in Fig. 1 is replaced with two unipolar switches 18a and 18b. In the embodiment of Fig. 9, a splitter 72 is arranged between the light source 28 and the optical switches 18a and 18b, which may be a 50-50 splitter.

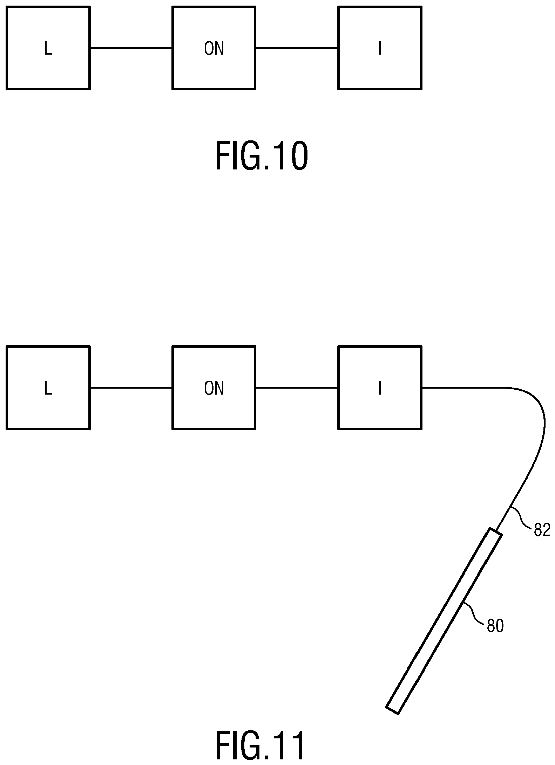

The optical network 10 based on PDSC can be used in any OFDR systems. Fig. 10 shows a sketch of an OFDR system, comprising a light source L like light source 28, in particular a tunable laser, an optical network ON according to the teachings of the present disclosure, and an interferometer I. The light source L may be configured to trigger the at least one optical switch 18 or the optical switches 18a, 18b of the optical network ON.

PDSC can be used as a modular hardware component as part of the optical network of a FORS system, thereby making the FORS system more robust, more accurate and of lower cost. Fig. 11 shows an embodiment of an FORS system which may also be referred to as optical shape sensing system, which comprises the light source L, in particular a tunable laser, the optical network ON according to the teachings of the present disclosure, the interferometer I and a shape sensing enabled device 80, e.g. a catheter or a guidewire equipped with an optical fiber 82 connected to the interferometer I. The optical network ON according to the teachings of the present disclosure may form part of the interrogator of the FORS system.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1.    Optical network, comprising:

a fiber optic path (12) comprising:

a first fiber optic branch (14) and a second fiber optic branch (16) separate from the first fiber optic branch (14), at least one optical switch (18; 18a, 18b) to toggle between input of light into the first fiber optic branch (14) and input into the second fiber optic branch (16), a polarization beam combiner (20) on an output side of the first and second fiber optic branches (14, 16), at least one polarization controller (24, 26; 70) for controlling polarization in the fiber optic path (12),

an optical power measuring modality (32) for measuring the optical output power of the polarization beam combiner (20).

2.    Optical network of claim 1, wherein the at least one polarization controller (24, 26; 70) is arranged in one of the first and second fiber optic branches (14, 16) or upstream of the optical switch (18).

3.    Optical network of claim 1, wherein the at least one polarization controller (24, 26) comprises a first polarization controller (24) arranged in the first fiber optic branch (14) and a second polarization controller (26) arranged in the second fiber optic branch (16).

4.    Optical network of claim 1, wherein the at least one polarization controller (24, 26) comprises a first polarization controller (24; 26) arranged in one of the first and second fiber optic branches (14, 16), and a second polarization controller (70) arranged upstream of the optical switch (18).

5.    Optical network of claim 1, wherein the at least one optical switch (18; 18a, 18b) is a MEMS-based optical switch.

6.    Optical network of claim 1, wherein the at least one optical switch (18) comprises one bipolar optical switch connecting to both the first and second fiber optic branches (14, 16).

7.    Optical network of claim 1, wherein the at least one optical switch (18a, 18b) comprises a first unipolar optical switch (18a) connecting to the first fiber optic branch (14) and a second unipolar optical switch (18b) connecting to the second fiber optic branch (16).

8. Optical network of claim 1, wherein the optical switch (18; 18a, 18b) is synchronized with wavelength scans of light emitted by a light source (28) such that the wavelength scans alternate at the output of the polarization beam combiner (20).

9. Optical network of claim 1, wherein the at least one polarization controller (24, 26; 70) is configured to set the polarization based on the measured output power of the polarization beam combiner (20).

10. Optical network of claim 1, wherein the optical power measuring modality (32) is configured to control the at least one polarization controller (24, 26; 70).

11. System for Optical Frequency Domain Reflectometry, comprising:

a light source (L) for emitting light, and
an optical network (ON) according to anyone of claims 1 to 9 into which light emitted by the light source (28) is fed.

12. System of claim 11, wherein the light source (L) is configured to trigger the at least one optical switch (18; 18a, 18b) according to wavelength scans of the light.

13. Optical shape sensing system, comprising a system according to claim 11 and an elongated optical fiber (82) connected to the optical network (ON) for interrogating the optical fiber (82).

14. Method of creating time-multiplexed orthogonal polarization states of light in a fiber optic network (10), comprising:

inputting a light beam into a fiber optic path (12), the fiber optic path (12) comprising a first fiber optic branch (14) and a second fiber optic branch (16) separate from the first fiber optic branch (14),
toggling between input of light into the first fiber optic branch (14) and input of light into the second fiber optic branch (16),
controlling polarization of the light in the fiber optic path (12),
inputting the light from the first and second fiber optic branches (14, 16) into a polarization beam combiner (20),
measuring an optical output power of the polarization beam combiner (20).

FIG.1

FIG.2

Transmission

FIG.3a

SOP

FIG.3b

FIG.4

FIG.5a

FIG.5b

FIG.6

FIG.7

FIG.8

FIG.9

EP 4 467 957 A1

L — ON — I

## FIG.10

L — ON — I — 82 / 80

## FIG.11

Europäisches
Patentamt

European
Patent Office

Office européen
des brevets

# EUROPEAN SEARCH REPORT

Application Number

**EP 23 17 5071**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/164627 A1 (FROGGATT MARK E [US] ET AL) 27 July 2006 (2006-07-27) <br> * paragraph [0016] - paragraph [0017] * <br> * paragraph [0022] * <br> * paragraph [0031] - paragraph [0032] * <br> * paragraph [0044] * <br> * paragraph [0047] * <br> * figure 4 * <br> ----- | 1-14 | INV. <br> G01M11/00 <br> A61B34/20 <br> G01B11/16 <br> H04B10/00 <br> H04J14/00 |
| Y | US 2014/140691 A1 (REAVES MATTHEW T [US] ET AL) 22 May 2014 (2014-05-22) <br> * paragraphs [0005], [0006], [0007] * <br> * paragraph [0054] - paragraph [0066] * <br> * figure 8 * <br> ----- | 1-14 | |
| A | US 9 009 003 B1 (CHAN HON MAN [US] ET AL) 14 April 2015 (2015-04-14) <br> * figures 2-4 * <br> ----- | 1-14 | |
| A | US 2022/182141 A1 (KREGER STEPHEN T [US] ET AL) 9 June 2022 (2022-06-09) <br> * paragraph [0003] * <br> * paragraph [0043] - paragraph [0044] * <br> * paragraph [0049] * <br> ----- | 1,14 | TECHNICAL FIELDS SEARCHED (IPC) <br> <br> G01M |
| A | US 2015/263804 A1 (HORIKX JEROEN JAN LAMBERTUS [NL] ET AL) 17 September 2015 (2015-09-17) <br> * paragraphs [0033], [0036], [0046], [0047] * <br> ----- | 1,14 | |

-/--

The present search report has been drawn up for all claims

| Place of search | Date of completion of the search | Examiner |
|---|---|---|
| The Hague | 11 October 2023 | Votini, Stefano |

EPO FORM 1503 03.82 (P04C01)

Europäisches
Patentamt

European
Patent Office

Office européen
des brevets

# EUROPEAN SEARCH REPORT

Application Number

EP 23 17 5071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NIANYU ZOU ET AL: "Measurement of polarization mode dispersion based on optical frequency domain reflectometry technique", OPTICAL FIBER COMMUNICATION CONFERENCE. (OFC). TECHNICAL DIGEST POSTCONFERENCE EDITION. ANAHEIM, CA, MARCH 17 - 22, 2001; [TRENDS IN OPTICS AND PHOTONICS SERIES. TOPS. VOLUME 54], WASHINGTON, WA : OSA, US, 17 March 2001 (2001-03-17), page ThA1, XP031969037, ISBN: 978-1-55752-655-7 * abstract * * figure 2 * | 1-14 | |
| A | US 2005/121633 A1 (ZIEGLER PATRICK [DE] ET AL) 9 June 2005 (2005-06-09) * paragraph [0001] * * paragraph [0022] * * figures 1-2 * | 1,14 | |
| | | | TECHNICAL FIELDS SEARCHED (IPC) |
| A | FAN X ET AL: "Full polarimetric phase-noise-compensated optical-frequency-domain reflectometry for distributed measurement of high-PMD fibers", OPTICS LETTERS, OPTICAL SOCIETY OF AMERICA, US, vol. 35, no. 1, 1 January 2010 (2010-01-01), pages 25-27, XP001551101, ISSN: 0146-9592, DOI: 10.1364/OL.35.000025 * figure 1a * * abstract * | 1,14 | |
| | -/-- | | |

The present search report has been drawn up for all claims

| Place of search | Date of completion of the search | Examiner |
|---|---|---|
| The Hague | 11 October 2023 | Votini, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUTTNER B ET AL: "Local birefringence measurements in single-mode fibers with coherent optical frequency-domain reflectometry", IEEE PHOTONICS TECHNOLOGY LETTERS, IEEE, USA, vol. 10, no. 10, 1 October 1998 (1998-10-01), pages 1458-1460, XP011425740, ISSN: 1041-1135, DOI: 10.1109/68.720293 * abstract * | 1,14 | |
| A | ITO FUMIHIKO ET AL: "Long-range coherent optical frequency domain reflectometry and its applications", 21ST INTERNATIONAL CONFERENCE ON OPTICAL FIBER SENSORS, SPIE, 1000 20TH ST. BELLINGHAM WA 98225-6705 USA, vol. 7753, no. 1, 15 May 2011 (2011-05-15), pages 1-4, XP060011625, DOI: 10.1117/12.884820 [retrieved on 1901-01-01] * figure 1 * | 1,14 | TECHNICAL FIELDS SEARCHED (IPC) |
| A | US 2017/276523 A1 (LALLY EVAN M [US] ET AL) 28 September 2017 (2017-09-28) * figure 1 * | 1,14 | |

-/--

The present search report has been drawn up for all claims

| Place of search | Date of completion of the search | Examiner |
|---|---|---|
| The Hague | 11 October 2023 | Votini, Stefano |

Europäisches
Patentamt

European
Patent Office

Office européen
des brevets

# EUROPEAN SEARCH REPORT

Application Number

EP 23 17 5071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XINYU FAN ET AL: "2-cm spatial resolution over 40 km realized by bandwidthdivision phase-noise-compensated OFDR", 2011 OPTICAL FIBER COMMUNICATION CONFERENCE AND EXPOSITION AND THE NATIONAL FIBER OPTIC ENGINEERS CONFERENCE (OFC/NFOEC 2011) : LOS ANGELES, CALIFORNIA, USA, 6 - 10 MARCH 2011, IEEE, PISCATAWAY, NJ, USA, 6 March 2011 (2011-03-06), pages 1-3, XP031946211, ISBN: 978-1-4577-0213-6 * abstract * ----- | 1,14 | |
| A | WILLIAMS P A ET AL: "Direct Measurement of Vector Polarization-Mode Dispersion From Repeated Random Data by Use of Linear Optical Sampling", IEEE PHOTONICS TECHNOLOGY LETTERS, IEEE, USA, vol. 22, no. 5, 1 March 2010 (2010-03-01), pages 326-328, XP011287448, ISSN: 1041-1135 * figure 1 * ----- | 1,14 | |
| A | JP 2015 190917 A (ANRITSU CORP) 2 November 2015 (2015-11-02) * paragraph [0060] - paragraph [0068] * ----- | 1,14 | TECHNICAL FIELDS SEARCHED (IPC) |
| A | WO 03/054595 A1 (PROXIMION FIBER OPTICS AB [SE]; NYHOLM LEIF [SE]; SAHLGREN BENGT [SE]) 3 July 2003 (2003-07-03) * figure 1 * ----- | 1,14 | |

The present search report has been drawn up for all claims

| Place of search | Date of completion of the search | Examiner |
|---|---|---|
| The Hague | 11 October 2023 | Votini, Stefano |

EPO FORM 1503 03.82 (P04C01)

## ANNEX TO THE EUROPEAN SEARCH REPORT
## ON EUROPEAN PATENT APPLICATION NO.

EP 23 17 5071

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2023

| Patent document cited in search report | | | Publication date | Patent family member(s) | | | Publication date |
|---|---|---|---|---|---|---|---|
| US | 2006164627 | A1 | 27-07-2006 | AU | 2003256461 | A1 | 23-01-2004 |
| | | | | US | 2006164627 | A1 | 27-07-2006 |
| | | | | WO | 2004005973 | A2 | 15-01-2004 |
| US | 2014140691 | A1 | 22-05-2014 | EP | 2727264 | A2 | 07-05-2014 |
| | | | | US | 2014140691 | A1 | 22-05-2014 |
| | | | | WO | 2013003141 | A2 | 03-01-2013 |
| US | 9009003 | B1 | 14-04-2015 | NONE | | | |
| US | 2022182141 | A1 | 09-06-2022 | EP | 3948205 | A1 | 09-02-2022 |
| | | | | US | 2022182141 | A1 | 09-06-2022 |
| | | | | WO | 2020214637 | A1 | 22-10-2020 |
| US | 2015263804 | A1 | 17-09-2015 | CN | 104782063 | A | 15-07-2015 |
| | | | | EP | 2720388 | A1 | 16-04-2014 |
| | | | | EP | 2907249 | A1 | 19-08-2015 |
| | | | | JP | 5829784 | B1 | 09-12-2015 |
| | | | | JP | 2015536445 | A | 21-12-2015 |
| | | | | US | 2015263804 | A1 | 17-09-2015 |
| | | | | WO | 2014060158 | A1 | 24-04-2014 |
| US | 2005121633 | A1 | 09-06-2005 | EP | 1468262 | A1 | 20-10-2004 |
| | | | | JP | 2005515428 | A | 26-05-2005 |
| | | | | US | 2005121633 | A1 | 09-06-2005 |
| | | | | WO | 03060458 | A1 | 24-07-2003 |
| US | 2017276523 | A1 | 28-09-2017 | US | 2017276523 | A1 | 28-09-2017 |
| | | | | WO | 2016029110 | A1 | 25-02-2016 |
| JP | 2015190917 | A | 02-11-2015 | JP | 6291313 | B2 | 14-03-2018 |
| | | | | JP | 2015190917 | A | 02-11-2015 |
| WO | 03054595 | A1 | 03-07-2003 | AU | 2002366934 | A1 | 09-07-2003 |
| | | | | WO | 03054595 | A1 | 03-07-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004005973 A2